# FASCICULE DE BREVET EUROPEEN

(11) **EP 3 562 466 B1**
(45) Date de publication et mention de la délivrance du brevet: **03.08.2022**
(21) Numéro de dépôt: 17837981.4
(22) Date de dépôt: 26.12.2017
(51) Int. Cl.: A61K 8/34, A61K 8/60, A61Q 11/02, A61K 8/73, A61K 8/97, A61K 8/19, A61Q 11/00, A61K 8/04

(54) **GEL DE BLANCHIMENT DENTAIRE SUR BASE NATURELLE ET SANS PEROXYDES**
ZAHNAUFHELLUNGSGEL AUF NATÜRLICHER BASIS UND OHNE PEROXIDE
DENTAL WHITENING GEL MADE OF A NATURAL, PEROXIDE-FREE BASE

(30) Priorité: 28.12.2016 EP 16207176
(43) Date de publication de la demande: 06.11.2019
(73) Titulaire: Bochenek, Stéphane, 91330 Yerres (FR)
(72) Inventeur: Bochenek, Stéphane, 91330 Yerres (FR)
(86) Numéro de dépôt international: PCT/FR2017/053846
(87) Numéro de publication internationale: WO 2018/122529

(56) Documents cités:
- WO-A1-99/34773
- US-A1- 2010 303 737
- US-A1- 2015 322 374
- Anonyme: "FAQ - Questions fréquentes posées par nos internautes", BeconfiDent , 2013, XP002769967, Extrait de l'Internet: URL:http://www.beconfident.fr/questions-fr equentes [extrait le 2017-05-05]

## Description

La présente invention est relative à un gel de blanchiment dentaire dépourvu de peroxydes et de composés dérivés de la pétrochimie.

Le blanchiment dentaire vise à éclaircir la coloration des dents dans un but esthétique. Les défauts de coloration des dents peuvent être d'origine naturelle ou physiologique (par exemple liés au vieillissement de la dent), ou provenir de facteurs exogènes (par exemple prise de tétracycline pendant la formation de la dent, consommation importante de café, thé, ou tabac, etc.).

Divers types de traitements de blanchiment dentaires sont actuellement disponibles ; ils sont principalement basés sur l'utilisation de composés peroxydés (notamment peroxyde d'hydrogène ou peroxyde de carbamide) en tant qu'agents blanchissants. Ces agents de blanchiment peroxydés présentent, outre une bonne efficacité décolorante sur les pigments constitutifs des taches dentaires, une action bactéricide notable permettant de lutter contre les bactéries de la plaque dentaire.

Certains de ces traitements sont effectués en cabinet dentaire, en une ou plusieurs séances. Une gouttière épousant la forme de l'arcade dentaire, confectionnée sur mesure et remplie d'un produit de blanchiment très concentré en agent blanchissant est appliquée à la mâchoire à traiter pour une durée de pose de l'ordre de quelques dizaines de minutes, variable selon la coloration initiale de la dent. Le cas échéant, le processus d'éclaircissement peut être activé par l'emploi d'une lampe à laser. Ces traitements en cabinet peuvent être remplacés ou complétés par un traitement ambulatoire prescrit par le dentiste, utilisant les mêmes gouttières avec un produit de blanchiment plus faiblement dosé. Les gouttières sont appliquées quotidiennement pendant une durée variant de quelques minutes à une heure par jour, jusqu'à ce que l'éclaircissement souhaité soit atteint.

À côté des traitements qui sont destinés à être effectués sous la supervision d'un dentiste il existe également des traitements utilisables à domicile qui sont vendus sans prescription médicale sous forme de kits prêts à l'emploi. Comme les précédents, ces traitements font appel à des agents blanchissants à base de peroxydes, mais ceux-ci sont utilisés à des concentrations beaucoup plus faibles.

Ils se présentent sous différentes formes, associant une composition blanchissante à un dispositif applicateur. On citera notamment :
- les applicateurs sous forme de pinceau ou de stylo : ils permettent une application précise du produit blanchissant, pour une utilisation localisée sur quelques dents ;
- les bandes autocollantes recouvertes de composition blanchissante, qui peuvent être positionnées sur les dents à traiter
- les gouttières buccales préformées, pouvant être remplies de composition blanchissante.

Généralement, les compositions de blanchiment dentaire se présentent sous forme de gels permettant de maintenir un contact prolongé avec l'émail dentaire, et contiennent des gélifiants leur conférant la viscosité souhaitée. Elles contiennent également des humectants qui permettent une bonne humectation des gencives et un transfert efficace des agents actifs sur la surface des dents, et des émulsifiants qui assurent la mouillabilité du gel sur la surface de la dent et facilitent l'élimination facile des débris et pigments incrustés sur l'émail.

Malgré leur efficacité blanchissante et leurs propriétés bactéricides, l'utilisation d'agents de blanchiment peroxydés pose divers problèmes. En effet, il a été rapporté que les peroxydes pouvaient induire une augmentation de la sensibilité des dents chez certaines personnes, ainsi qu'une irritation gingivale. Le risque de tels effets indésirables est accru dans le cas des traitements vendus sans prescription médicale pour l'utilisation à domicile, car il est dans ce cadre difficile de contrôler si les modalités de traitement préconisées sont effectivement respectées. D'autre-part, c'est un procédé agressif qui nécessite absolument une reminéralisation après traitement sous peine d'obtenir l'effet inverse du résultat recherché ; en effet, les dents devenues "poreuses" vont absorber très rapidement les produits colorants (thé, café, tabac, vin, fruits, ...). Ces inconvénients ont conduit les législations de nombreux pays à limiter drastiquement la concentration maximale autorisée de peroxyde d'hydrogène dans les compositions de blanchiment dentaire destinées au grand public ; cette concentration maximale autorisée est, par exemple, de 0,1% en France.

Par ailleurs, la plupart des gels de blanchiment dentaire présents sur le marché utilisent des carbomères comme agents de texture destinés à contrôler leur viscosité. Ces carbomères sont des polymères synthétiques hydrophiles d'acide acrylique. Ils permettant d'obtenir facilement l'apparence d'un gel aqueux et sont donc utilisés comme agents épaississants dans l'industrie cosmétique. La triéthanolamine ou l'aminométhyl propanol permet de les neutraliser et de stabiliser et contrôler la viscosité du gel.

Le gel de blanchiment BeconfiDent^{R} utilise le Polysorbate 20 comme agent de texture.

Bien qu'ils ne soient pas considérés comme toxiques dans le cadre d'une utilisation cosmétique, l'origine pétrochimique de ces composés, leur très faible biodégradabilité et leur capacité de remobiliser les ions calcium et magnésium font actuellement l'objet de critiques. Par ailleurs, ils ne figurent pas sur la liste des additifs alimentaires autorisés. Or, lors de l'utilisation d'un gel de blanchiment dentaire, une partie significative du produit peut être ingérée par déglutition.

La présente invention vise à obtenir de nouvelles compositions de blanchiment des dents à base naturelle, permettant de prévenir les problèmes liés à l'utilisation de dérivés peroxydés et de dérivés de la pétrochimie, tout en conservant les modalités d'emploi et les propriétés blanchissantes et bactéricides des compositions de l'art antérieur.

Dans ce but, la présente invention fournit des gels de blanchiment dentaire contenant en tant que seul agent blanchissant, du bicarbonate de sodium ou de potassium, assurant le blanchiment par interaction chimique avec les dépôts anthocyaniques ou polyphénoliques fixés sur l'émail ou la plaque dentaire. Cet agent blanchissant est associé à au moins un extrait végétal et/ou au moins une huile essentielle végétale présentant un caractère bactéricide ou bactériostatique.

L'incorporation d'extrait végétal et/ou d'huile essentielle a posé le problème de leur solubilisation. Cette solubilisation est usuellement effectuée par un prémélange avec un dérivé fortement éthoxylé de l'huile de ricin (par exemple huile de ricin hydrogénée éthoxylée à 40 unités d'oxyde d'éthylène). Or, l'éthoxylation est considérée comme un procédé polluant, et susceptible de générer, lors de sa mise en œuvre, du 1,4-dioxane, un contaminant classé potentiellement cancérigène. Cette solution ne pouvait donc être retenue.

En poursuivant leurs recherches les inventeurs ont constaté que des composés tensioactifs non ioniques de la classe des alkyl polyglucosides présentaient, de manière surprenante, des propriétés hydrosolubilisantes proches de celles des huiles de ricin éthoxylées, et permettaient de se dispenser de l'utilisation de celles-ci.

Les alkyl polyglucosides ont montré en outre d'excellentes propriétés détergentes permettant l'élimination des dépôts colorés sur l'émail des dents, sans irritation de la muqueuse des gencives, permettant leur utilisation comme seuls émulsifiants dans les compositions conformes à l'invention.

Afin d'éviter la présence de dérivés de la pétrochimie, les seuls gélifiants utilisés dans les gels de blanchiment dentaire de la présente invention, sont des polysaccharides, et les seuls humectants utilisés sont des polyols.

La présente invention a pour ainsi objet un gel de blanchiment dentaire comprenant :
- un agent blanchissant constitué par du bicarbonate de sodium ou de potassium ;
- un agent émulsifiant constitué par un ou plusieurs alkyl polyglucoside(s) ;
- un agent gélifiant constitué par un ou plusieurs polysaccharides ;
- un agent humectant constitué par un ou plusieurs polyols ;
- un agent bactériostatique ou bactéricide constitué par au moins un extrait végétal et/ou au moins une huile essentielle végétale.

Les polysaccharides gélifiants utilisés dans les compositions de l'invention sont d'origine végétale ou bactérienne. Il peut s'agir de polysaccharides naturels, ou de polysaccharides modifiés ou semi-synthétiques dérivés des précédents.

Les polysaccharides d'origine végétale utilisables dans le cadre de la présente invention peuvent être issus d'algues. On citera notamment les alginates, les carraghénanes et l'agar-agar.

Les alginates sont des polysaccharides extraits d'algues brunes, notamment des fucales ou des laminariales. Les carraghénanes sont des polysaccharides obtenus à partir d'algues rouges, notamment des Solieriacées, Gigartinacées, Furcellariacées, Hypneacées, Rhabdoniacées et Rhodophyllidacées. L'agar-agar est extrait de la paroi cellulaire de certaines espèces d'algues rouges, notamment des Gelidiacées et Gracilariacées. L'agarose purifié à partir de l'agar agar peut également être utilisé dans le cadre de la présente invention.

D'autres polysaccharides d'origine végétale utilisables dans le cadre de la présente invention sont obtenus à partir de végétaux terrestres. Il s'agit notamment de gommes végétales (par exemple gomme de caroube, gomme tara, gomme de guar, gomme arabique, gomme ghatti), des pectines, des amidons, ainsi que des dérivés semi-synthétiques de cellulose ou d'amidons.

La gomme de caroube, la gomme de guar, et la gomme tara sont respectivement extraites des graines de *Ceratonia siliqua,* de *Cyamopsis tetragonoloba* et de *Caesalpinia spinosa.*

La gomme arabique et la gomme ghatti sont produites à partir d'exsudations de blessures du tronc de certaines espèces d'acacias (notamment *Acacia senegal*), dans le cas de la gomme arabique, et *d'Anogeissus latifolia* dans celui de la gomme ghatti.

Les pectines sont principalement produites à partir de parois végétales de fruits (pommes, agrumes, etc), qui sont des sous-produits de l'industrie du jus de fruits. L'amidon est produit à partir des tissus de réserve de plantes très diverses (maïs, manioc, pomme de terre, etc).

Des dérivés semi-synthétiques ou modifiés de polysaccharides végétaux qui peuvent être utilisés dans le cadre de l'invention sont par exemple la carboxyméthyl cellulose, l'hydroxyéthylcellulose, l'hydroxyéthyl-méthyl-cellulose, les amidons modifiés, etc....

Des polysaccharides d'origine bactérienne utilisables dans les compositions de la présente invention sont notamment la gomme gellane produite par *Sphingomonas elodea*, et la gomme xanthane produite par *Xanthomonas campestris.*

Selon le mode d'application envisagé pour le gel de blanchiment dentaire, sa texture et sa viscosité peuvent être ajustées notamment par la nature et la concentration de gélifiants et le pH de la solution, afin d'obtenir une restitution facile à partir du système de distribution en même temps qu'une tenue suffisante sur la surface dentaire. Avantageusement, on pourra combiner entre eux deux ou plusieurs polysaccharides gélifiants. On considère généralement une viscosité comprise entre 40 000 et 150 000 mPa.s comme optimale

L'agent humectant utilisé dans les compositions de l'invention est constitué de préférence par un polyol ou un mélange de polyols.

À titre d'exemples, on peut citer : le glycérol, le sorbitol, l'érythritol, le pentaérythritol, le xylitol, le mannitol, l'arabitol, le maltitol et l'isomaltitol.

On peut également utiliser des oses hémiacétalysés (sucres cyclisés) non cariogènes : des monosaccharides, tels que le fructose, le galactose, le mannose, etc.... ou des disaccharides, tels que le lactose ou le maltose, etc....

Dans une composition préférée, on associera le glycérol, le sorbitol et le xylitol, ce dernier ayant d'une part un effet reconnu de rafraîchissement de la bouche et d'autre part d'inhibition de la production d'acides dans la plaque dentaire et de diminution d'adhésivité et la quantité de la plaque, la rendant plus aisée à enlever à la brosse.

Le gel de blanchiment dentaire conforme à l'invention comprend en outre un mélange d'extraits végétaux obtenus à partir de plantes possédant des actions bactéricide ou bactériostatique, se substituant ainsi à l'action bactéricide des agents peroxydants. Ces extraits assurent la conservation du produit de manière naturelle, ont un effet inhibiteur sur le développement de la plaque dentaire

Il peut s'agir par exemple d'une ou plusieurs huile(s) essentielle(s) ou extrait(s) hydro-alcoolique(s) ou hydro-glycolique(s) connu(e)s pour leurs propriétés bactéricides ou bactériostatiques et utilisable(s) dans les produits d'hygiène dentaire.

À titre indicatif, on peut citer les huiles essentielles ou extraits de Bois de Rose, Cajeput, Écorce de Cannelle, Citron, Citron vert, Citronnelle de Madagascar, Copahu, Curcuma, *Eucalyptus Globulus,* Eucalyptus Radié, Géranium Rosat, Girofle, Bois de Hô, Laurier noble, Lavande Aspic, Lavande Fine, Manuka, Marjolaine à Thujanol, Menthe Bergamote, Menthe Poivrée, Menthe Verte, Niaouli, Origan, Palmarosa, Pin Sylvestre, Pruche, Ravintsara, Sapin Baumier, Sapin Blanc Argenté, Sapin de Sibérie, Sarriette, *Melaleucea alternifolia* (Arbre à thé), Thym à Linalol, Thym Satureoides à Bornéol, etc....

Les alkyl polyglucosides qui sont utilisés comme émulsifiant dans les compositions de l'invention sont des tensioactifs non ioniques d'origine végétale, obtenus à partir d'amidon et d'alcools gras.

Des alkyl polyglucosides préférés sont définis par la formule : 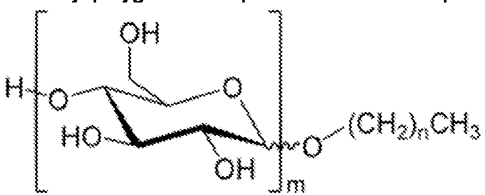 où n est un entier compris entre 7 et 17, de préférence entre 9 et 15, et m (nombre d'unités glucose) est un entier compris entre 1 et 5.

On peut citer à titre d'exemples non limitatifs de cette famille de composés : le Plantacare 818 UP de la société BASF (cocoyl glucoside), le Plantacare 810 de la société BASF (caprilyl/capryl glucoside), le Triton CG-110 de la société Dow, etc....

La composition objet de l'invention peut en outre comprendre des extraits végétaux ou des huiles essentielles présentant des propriétés bénéfiques sur la muqueuse gingivale telles que propriétés adoucissantes, anti- inflammatoires et/ou anti-oxydantes. Cette fraction d'extraits végétaux comprend, par exemple, un mélange d'extrait de feuille d'aloès, d'extrait de grenade, et d'extrait de fleurs de camomille.

Dans une composition préférée, un gel de blanchiment dentaire selon l'invention peut comprendre en outre un extrait végétal, obtenu à partir la plante *Salvadora Persica* (également nommée miswak ou siwak). Cet extrait d'un arbuste de la famille des Salvadoraceae, originaire du Moyen-Orient est un inhibiteur bactérien empêchant les bactéries de constituer la plaque dentaire et permet d'éviter l'halitose d'origine bactérienne buccale. Il contient par ailleurs de la thio-urée et des dérivés soufrés de carbamide, ainsi que des alcaloïdes dérivés de thiocarbamide, et de carbamide qui donnent au *Salvadora Persica* une excellente efficacité au niveau du blanchiment de la denture. Ces extraits se présentent en général sous forme de poudre non hydrosolubles ou de suspension aqueuse ; ils doivent donc être incorporés dans des compositions de viscosité supérieure à 5 000 mPa.s ou à effet de seuil notable, afin d'assurer la stabilité de leur maintien en suspension ; leur concentration reste en général inférieure à 0,5% afin de ne pas affecter significativement l'esthétique du gel.

Généralement, un gel de blanchiment dentaire selon l'invention comprendra également un ou plusieurs agent(s) aromatisant(s), permettant de rendre son utilisation plus agréable pour le consommateur. Il peut s'agir de tout arôme utilisable dans les produits d'hygiène dentaire. Il peut comprendre également un ou plusieurs colorants, dont la nature dépendra de la couleur que l'on souhaite donner à la préparation.

Habituellement, un gel de blanchiment dentaire selon l'invention comprendra :
- entre 15 et 50%, préférentiellement entre 20 et 35 % en poids d'une solution aqueuse saturée à 9% de bicarbonate de sodium ;
- entre 40 et 70 % en poids d'agent humectant ; si cet agent humectant est un mélange de glycérol, de sorbitol et de xylitol, ce mélange comprendra avantageusement de 98,8 % à 99,8 % en poids de glycérol, de 0,2 à 1% en poids de sorbitol, et de 0,05 à 0,2 % en poids de xylitol.
- entre 0,5 et 5 % en poids de polysaccharide gélifiant comprenant de préférence entre 1 et 1,5% en poids de gomme xanthane ou gellane, et entre 0,1 et 0,5% en poids de carraghénane ;
- entre 0,05 % et 0,5 % en poids d'un ou plusieurs alkyl polyglucosides, caractérisés en ce que leur chaîne alkyle comprend de 8 à 18 atomes de carbone, et leur chaîne glucoside de 1 à 5 unités de glucose.
- entre 0,05 % et 0,5 % en poids d'huile(s) essentielle(s) ou extrait(s) hydroalcoolique(s) ou hydro-glycolique(s) de plantes connues pour leurs propriétés bactéricides ou bactériostatiques et utilisables dans les produits d'hygiène dentaire
- le complément éventuel à 100% en eau déionisée

Lorsque des arômes et/ou des colorants sont utilisés, leur dosage dépendra principalement de leur nature, ainsi que de l'intensité du goût et/ou de la couleur que l'on souhaite conférer au produit final. À titre d'exemple, si l'on utilise de l'huile de menthe poivrée à titre d'arôme, elle sera utilisée à raison de 0,05 à 0,2 % en poids du produit final.

La présente invention a également pour objet un procédé de préparation d'un gel de blanchiment dentaire selon l'invention. Ce procédé comprend les étapes suivantes :
a. la préparation du polyol ou du mélange de polyols utilisé comme agent humectant ;
b. l'incorporation audit agent humectant du mélange de polysaccharides gélifiants, sous agitation constante jusqu'à complète dispersion et formation d'un gel transparent et homogène;
c. le maintien du mélange obtenu à l'étape b) sous agitation intermittente pendant 12 à 48 heures ;
d. l'addition du bicarbonate de sodium en solution dans l'eau préalablement préchauffée à une température inférieure à 60°C, sous agitation constante jusqu'à obtention d'un gel transparent et homogène.
e. l'addition sous agitation d'un prémélange constitué de (des) alkyl polyglucoside(s) d'origine végétale légèrement chauffé(s) (35°-45°C) et homogénéisé(s) avec l'(les) huile(s) essentielle(s) ou extrait(s) végétal(aux) bactéricide(s) ou bactériostatique(s) ; ledit mélange étant, par exemple, dans des proportions alkyl polyglucoside / extrait végétal de l'ordre de 1:1 à 4:1.

Si le gel de blanchiment dentaire contient un autre extrait de plante(s), cet extrait est ajouté, sous agitation, au gel obtenu à l'issue de l'étape e) et l'agitation est poursuivie pour permettre son incorporation.

Si l'on souhaite ajouter des arômes et/ou des colorants, ceux-ci sont ajoutés, toujours sous agitation, après l'extrait de plante, et l'agitation est poursuivie pour permettre leur incorporation au mélange.

La présente invention a également pour objet un procédé esthétique de blanchiment des dents caractérisé en ce qu'il comprend l'application aux dents dont l'aspect est à améliorer d'un gel de blanchiment dentaire selon l'invention.

Généralement, le gel de blanchiment dentaire selon l'invention sera utilisé en applications quotidiennes ou biquotidiennes, d'au moins 5 minutes, et de préférence de 10 à 60 minutes. Toutefois, la fréquence des applications, et la durée de chaque application peuvent, si besoin est, être augmentées ou diminuées. Les applications pourront être poursuivies jusqu'à ce que le degré d'éclaircissement désiré soit atteint. Du fait de l'absence de peroxydes, un traitement prolongé ne fait pas courir de risque de sensibilisation des dents ou d'irritation de la muqueuse gingivale.

La présente invention a également pour objet un kit pour le blanchiment des dents, comprenant un gel de blanchiment dentaire selon l'invention, et un dispositif permettant l'application dudit gel.

Le dispositif applicateur peut être par exemple sous forme de pinceau, de stylo ou de bâtonnet muni d'un embout de mousse, il peut s'agir également d'une bande autocollante enduite du gel de blanchiment selon l'invention, ou d'une gouttière buccale pouvant être remplie avec ledit gel.

La présente invention sera mieux comprise à l'aide du complément de description qui va suivre, qui se réfère à un exemple non-limitatif illustrant la préparation d'un gel de blanchiment des dents conforme à l'invention.

### Exemple :

### Ingrédients :

Les ingrédients utilisés pour la préparation d'un exemple de formule de gel selon l'invention sont listés dans le tableau ci-dessous :

| Ingrédient | N° CAS | Pourcentage en poids |
|---|---|---|
| Glycérol | CAS 56-81-5 | 64.40% |
| Bicarbonate de sodium (Solution à 9%) | CAS 144-55-8 | 32% |
| Gomme xanthane | CAS 11138-66-2 | 2.0% |
| Carraghénane (Poudre de *Chondrus crispus*) | CAS 9000-07-1 | 0.5% |
| Sorbitol (Solution à 70% dans l'eau) | CAS 50-70-4 | 0.5% |
| Xylitol (Solution à 50% dans l'eau) | CAS 87-99-0 | 0.2% |
| Cocoyl glucoside | 141464-42-8 | 0.2% |
| Huile essentielle de sarriette *(Satureja montana)* | CAS 8006-90-4 | 0.1% |
| Extrait de grenade (*Punica qranatum*) | CAS 84961-57-9 | 0.05% |
| Extrait de camomille (*Chamomilla recutita*) | CAS 84082-60-0 | 0.05% |
| Colorant CI 42090 | CAS 3844-45-9 | (Quelques gouttes) |
| Colorant CI 19140 | CAS 1934-21-0 | (Quelques gouttes) |

Les ingrédients utilisés proviennent de SPECTRUM CHEMICAL, à l'exception des suivants :
Huile de menthe poivrée et extrait de grenade (NATURES FLAVORS)
Extrait de camomille (BOTANIC CHOICE)
Colorants CI 42090 et CI 19140
Pour la préparation du gel, le glycérol est placé dans un récipient en acier inoxydable. L'agitation est mise en route, et la vitesse réglée pour former un vortex. Du liquide de refroidissement est mis en circulation autour du réservoir pour réduire l'accumulation de chaleur, de manière à ce que la température de la préparation n'excède pas 40°C.

Les solutions de sorbitol (solution aqueuse à 70%) et de xylitol (solution aqueuse à 50%) sont ajoutées dans le vortex, en continuant à mélanger pendant au moins 10 minutes.

Les ingrédients en poudre (gomme xanthane et poudre de *Chondrus crispus)* sont ajoutés par saupoudrage dans le vortex et on continue à mélanger jusqu'à ce que les poudres soient complétement dispersées.

On mélange par intermittence pendant 2 jours en utilisant le protocole cidessus. Un gel épais se forme après gonflement des polymères.

Au jour 3, on ajoute la solution de bicarbonate de sodium tout en mélangeant à vitesse moyenne.

Une fois la consistance désirée atteinte, on ajuste la vitesse du mélangeur pour garder la préparation en mouvement, et on continue à mélanger pendant 5 à 10 minutes.

On introduit ensuite le prémélange de cocoyl glucoside préchauffé à 45°C et homogénéisé et l'huile essentielle bactéricide (par exemple huile essentielle de sarriette ou de thym) et on laisse sous agitation environ 20 minutes.

On ajoute l'extrait de grenade et l'extrait de camomille à la préparation et on poursuit le mélange pendant 3 à 5 minutes.

On ajoute enfin 7 gouttes de colorant CI 42090 et 1 goutte de colorant CI 19140 pour 5 litres de gel, et on continue à mélanger pendant 10 minutes.

## Revendications

1. Gel de blanchiment dentaire comprenant :
- un agent blanchissant constitué par du bicarbonate de sodium ou de potassium ;
- un agent émulsifiant constitué par un ou plusieurs alkyl polyglucoside(s) ;
- un agent gélifiant constitué par un ou plusieurs polysaccharides ;
- un agent humectant constitué par un ou plusieurs polyols ;
- un agent bactériostatique ou bactéricide constitué par au moins un extrait végétal et/ou au moins une huile essentielle végétale.

2. Gel de blanchiment dentaire selon la revendication 1, **caractérisé en ce que** :
- l'agent blanchissant est constitué par du bicarbonate de sodium ;
- l'agent émulsifiant comprend au moins du cocoyl polyglucoside. ;
- l'agent gélifiant comprend au moins un carraghénane et de la gomme xanthane ;
- l'agent humectant comprend au moins un polyol choisi parmi le glycérol, le sorbitol, et le xylitol, ou un mélange d'au moins deux desdits polyols ;
- l'agent bactériostatique ou bactéricide au moins un extrait de plante choisie parmi : Écorce de Cannelle, Citron, Citron vert, Citronnelle de Madagascar, *Eucalyptus globulus,* Girofle, Menthe Bergamote, Menthe Poivrée, Menthe Verte, Origan, Sarriette, *Melaleucea alternifolia* , Thym.

3. Gel de blanchiment dentaire selon une des revendications 1 ou 2, **caractérisé en ce qu'**il comprend en outre au moins un extrait d'une ou plusieurs plantes présentant des propriétés adoucissantes, anti- inflammatoires et/ou anti-oxydantes.

4. Gel de blanchiment dentaire selon la revendication 3, **caractérisé en ce que** ledit extrait de plantes comprend un mélange d'un extrait de grenade et d'un extrait de fleurs de camomille.

5. Gel de blanchiment dentaire selon la revendication 4, **caractérisé en ce que** ledit extrait de plante comprend en outre une suspension ou un extrait liquide de la plante *Salvadora persica*

6. Gel de blanchiment dentaire selon une des revendications 1 à 5, **caractérisé en ce que** l'agent humectant est un mélange de glycérol, de sorbitol et de xylitol.

7. Gel de blanchiment dentaire selon une des revendications 1 à 6, **caractérisé en ce qu'**il comprend :
- entre 15 et 50%, préférentiellement entre 20 et 35 % d'une solution à 9% de bicarbonate de sodium dans l'eau ;
- entre 40 et 70 % en poids d'agent humectant;
- entre 0,5 et 5 % en poids d'agent gélifiant ;
- entre 0,05 % et 0,5 % en poids d'agent émulsifiant ;
- entre 0,05 % et 0,5 % en poids d'agent bactériostatique ou bactéricide.

8. Gel de blanchiment dentaire selon une des revendications 1 à 7, **caractérisé en ce qu'**il contient en outre un ou plusieurs agent(s) aromatisant(s) et un ou plusieurs agent(s) colorant(s).

9. Procédé de préparation d'un gel de blanchiment dentaire selon une des revendications 1 à 8, **caractérisé en ce qu'**il comprend les étapes suivantes :
a. la préparation du polyol ou du mélange de polyols utilisé comme agent humectant ;
b. l'incorporation audit agent humectant de l'agent gélifiant, sous agitation constante jusqu'à complète dispersion et formation d'un gel transparent et homogène ;
c. le maintien du mélange obtenu à l'étape b) sous agitation intermittente pendant 12 à 48 heures ;
d. l'addition de l'agent blanchissant en solution dans l'eau préalablement préchauffée à une température inférieure à 60°C, sous agitation constante jusqu'à obtention d'un gel transparent et homogène ;
e. l'addition sous agitation d'un prémélange constitué de l'agent émulsifiant légèrement chauffé (30°-35°C) et homogénéisé avec l'agent bactéricide ou bactériostatique.

10. Procédé selon la revendication 9 **caractérisé en ce qu'**il comprend en outre :
f. l'addition, sous agitation, de l'extrait de plante(s) défini dans les revendications 3 à 5 au produit obtenu à l'issue de l'étape e)
g. optionnellement, l'addition, sous agitation, du ou des agent(s) aromatisant(s) et du ou des colorant(s).

11. Procédé non-thérapeutique esthétique de blanchiment des dents **caractérisé en ce qu'**il comprend l'application aux dents dont l'aspect est à améliorer d'un gel de blanchiment dentaire selon une des revendications 1 à 8.

12. Kit pour le blanchiment des dents, **caractérisé en ce qu'**il comprend un gel de blanchiment dentaire selon une des revendications 1 à 8, et un dispositif permettant l'application dudit gel aux dents dont l'aspect est à améliorer.

## Patentansprüche

1. Zahnaufhellungsgel einschließlich:
- ein Bleichmittel bestehend aus Natrium- oder Kaliumbicarbonat;
- einen Emulgator bestehend aus einem oder mehreren Alkylpolyglucosid(en);
- ein Geliermittel bestehend aus einem oder mehreren Polysacchariden;
- ein Feuchthaltemittel bestehend aus einem oder mehreren Polyolen;
- ein bakteriostatisches oder bakterizides Mittel bestehend aus mindestens einem Pflanzenextrakt und/oder mindestens einem ätherischen Pflanzenöl.

2. Zahnaufhellungsgel nach Anspruch 1, **dadurch gekennzeichnet, dass**:
- der Aufheller besteht aus Natriumbicarbonat;
- der Emulgator umfasst mindestens Cocoylpolyglucosid.;
- das Geliermittel umfasst mindestens ein Carrageenan und Xanthangummi;
- das Feuchthaltemittel umfasst mindestens ein Polyol ausgewählt aus Glycerin, Sorbit und Xylit oder eine Mischung von mindestens zwei der Polyole;
- das bakteriostatische oder bakterizide Mittel mindestens einen Pflanzenextrakt ausgewählt aus: Zimtrinde,
Zitrone, Limette, Madagaskar-Zitronengras, Eukalyptus Globulus, Gewürznelke, Bergamotte-Minze, Pfefferminze,
Grüne Minze, Oregano, Bohnenkraut, Melaleuca Alternifolia, Thymian.

3. Zahnaufhellungsgel nach einem der Ansprüche 1 oder 2, **dadurch gekennzeichnet, dass** es weiterhin mindestens einen Extrakt aus einer oder mehreren Pflanzen enthält, die weichmachende, entzündungshemmende und antioxidative Eigenschaften aufweisen.

4. Zahnaufhellungsgel nach Anspruch 3, **dadurch gekennzeichnet, dass** der Pflanzenextrakt eine Mischung aus einem Granatapfelextrakt und einem Blütenextrakt von Kamille.

5. Zahnaufhellungsgel nach Anspruch 4, **dadurch gekennzeichnet, dass** das Pflanzenextrakt umfasst weiterhin eine Suspension oder einen Flüssigextrakt der Salvadora Persica-Pflanze

6. Zahnaufhellungsgel nach einem der Ansprüche 1 bis 5, **dadurch gekennzeichnet dass** das Feuchthaltemittel eine Mischung aus Glycerin, Sorbit und Xylit ist.

7. Zahnaufhellungsgel nach einem der Ansprüche 1 bis 6, **dadurch gekennzeichnet, dass** es enthält:
- zwischen 15 und 50 %, vorzugsweise zwischen 20 und 35 % einer 9 % Lösung von Natriumbicarbonat in Wasser;
- zwischen 40 und 70 Gew.-% Feuchthaltemittel;
- zwischen 0,5 und 5 Gew.-% Geliermittel;
- zwischen 0,05 und 0,5 Gew.-% Emulgator;
- zwischen 0,05 und 0,5 Gew.-% bakteriostatisches oder bakterizides Mittel.

8. Zahnaufhellungsgel nach einem der Ansprüche 1 bis 7, **dadurch gekennzeichnet** das ferner einen oder mehrere Geschmacksstoffe und einen oder mehrere Farbstoffe enthält.

9. Verfahren zur Herstellung eines Zahnaufhellungsgels nach einem der Ansprüche 1 bis 8, **dadurch gekennzeichnet, dass** es die folgenden Schritte umfasst:
a) die Herstellung des verwendeten Polyols oder Gemisches von Polyolen als Feuchthaltemittel;
b) einarbeiten des Geliermittels in das Feuchthaltemittel unter konstantem Rühren bis zur vollständigen Dispersion und Bildung eines transparenten und homogenen Gels;
c) halten der in Schritt (b) erhaltenen Mischung unter intermittierendem Rühren für 12 bis 48 Stunden;
d) die Zugabe des Bleichmittels in Lösung in Wasser, das zuvor auf eine Temperatur unter 60 °C vorgewärmt wurde, unter ständigem Rühren, bis ein transparentes und homogenes Gel erhalten wird;
e) die Zugabe unter Rühren einer Vormischung bestehend aus dem Emulgator leicht erhitzt (30°-35°C} und homogenisiert mit dem bakteriziden oder bakteriostatischen Mittel.

10. Verfahren nach Anspruch 9, **dadurch gekennzeichnet, dass** es ferner umfasst:
f. die Zugabe des Pflanzenextrakts (der Pflanzenextrakte) nach den Ansprüchen 3 bis 5 unter Rühren zu dem am Ende von Schritt (e) erhaltenen Produkt
g. gegebenenfalls die Zugabe des/der Aromastoffe(s) und des/der Farbstoff(e) unter Rühren.

11. Ästhetischer nicht-therapeutischer Prozess Zahnaufhellung, **dadurch gekennzeichnet, dass** sie umfasst die Anwendung eines Zahnaufhellungsgels nach einem der Ansprüche 1 bis 8 auf Zähne, deren Aussehen verbessert werden soll.

12. Kit zur Zahnaufhellung, **dadurch gekennzeichnet, dass** es ein Gel enthält von Zahnaufhellung nach einem der Ansprüche 1 bis 8 und eine Vorrichtung zur Anwendung des Gels auf Zähne, deren Aussehen verbessert werden soll.

## Claims

1. Tooth whitening gel including:
- a bleaching agent consisting of sodium or potassium bicarbonate;
- an emulsifying agent consisting of one or more alkyl polyglucoside(s);
- a gelling agent consisting of one or more polysaccharides;
- a humectant consisting of one or more polyols;
- a bacteriostatic or bactericidal agent consisting of at least one plant extract, or at least one plant essential oil.

2. Tooth whitening gel according to claim 1, **characterized in that**:
- the whitening agent consists of sodium bicarbonate;
- the emulsifying agent comprises at least cocoyl polyglucoside .;
- the gelling agent comprises at least one carrageenan and xanthan gum;
- the humectant comprises at least one polyol chosen from glycerol, sorbitol and xylitol, or a mixture of at least two of said polyols;
- the bacteriostatic or bactericidal agent comprises at least one plant extract chosen from: Cinnamon bark, Lemon, Lime, Madagascar lemongrass, Eucalyptus Globulus, Clove, Bergamot mint, Peppermint, Green mint, Oregano, Savory, Melaleuca Alternifolia, Thyme.

3. Dental whitening gel according to one of claims 1 or 2, **characterized in that** it further comprises at least one extract of one or more plants exhibiting softening, anti-inflammatory and anti-oxidant properties.

4. Dental whitening gel according to claim 3, **characterized in that** said plant extract comprises a mixture of a pomegranate extract and an extract of chamomile flowers.

5. Tooth whitening gel according to claim 4, **characterized in that** said plant extract further comprises a suspension or a liquid extract of the Salvadora Persica plant.

6. Tooth whitening gel according to one of claims 1 to 5, **characterized in that** the humectant is a mixture of glycerol, sorbitol and xylitol.

7. Tooth whitening gel according to one of claims 1 to 6, **characterized in that** it comprises:
- between 15 and 50%, preferably between 20 and 35% of a 9% solution of sodium bicarbonate in water;
- between 40 and 70% by weight of humectant;
- between 0.5 and 5% by weight of gelling agent;
- between 0.05% and 0.5% by weight of emulsifying agent;
- between 0.05% and 0.5% by weight of bacteriostatic or bactericidal agent.

8. Tooth whitening gel according to one of claims 1 to 7, **characterized in that** it further contains one or more flavoring agent(s) and one or more dying agent(s)

9. Process for preparing a tooth whitening gel according to one of the claims 1 to 8, **characterized in that** it comprises the following steps:
a. preparing the polyol or mixture of polyols used as a humectant;
b. incorporating the gelling agent into said humectant, with constant stirring until complete dispersion and formation of a transparent and homogeneous gel;
c. maintaining the mixture obtained in step (b) with intermittent stirring for 12 to 48 hours;
d. addition of the bleaching agent in solution in water preheated beforehand to a temperature below 60 ° C., with constant stirring until a transparent and homogeneous gel is obtained;
e. addition with stirring of a premix consisting of the emulsifying agent slightly heated (30°C - 35°C} and homogenized with the bactericidal or bacteriostatic agent.

10. Method according to claim 9 **characterized in that** it further comprises:
f. the addition, with stirring, of the plant extract(s) defined in claims 3 to 5 to the product obtained at the end of step (e)
g. optionally, the addition, with stirring, of the flavoring agent(s) and of the dye(s).

11. Aesthetic non-therapeutic teeth whitening process **characterized in that** it comprises the application of a dental whitening gel, according to one of claims 1 to 8, to teeth whose appearance is to be improved.

12. Kit for teeth whitening, **characterized in that** it comprises a tooth whitening gel according to one of claims 1 to 8, and a device for the application of said gel to teeth whose appearance is to be improved.
